# EUROPEAN PATENT APPLICATION

(11) **EP 3 892 267 A1**
(43) Date of publication of application: **13.10.2021**
(21) Application number: 20315147.7
(22) Date of filing: 09.04.2020
(51) Int. Cl.: A61K 31/165, A61K 31/17, A61K 31/175, A61K 31/197, A61K 31/198, A61K 31/352, A61K 31/353, A61K 31/357, A61K 31/433, A61K 31/436, A61K 31/473, A61K 31/501, A61K 31/519, A61K 31/53, A61P 35/00

(54) **COMBINATORY TREATMENT FOR LYMPHOMA**

(71) Applicant: Université de Paris, 75006 Paris (FR); Institut National de la Santé et de la Recherche Médicale, 75013 Paris (FR); ASSISTANCE PUBLIQUE, HOPITAUX DE PARIS, 75004 Paris (FR)
(72) Inventor: Baud, Véronique, 75013 Paris (FR); Thieblemont, Catherine, 75011 Paris (FR)
(74) Representative: A.P.I. Conseil

(57) **Abstract**

The invention relates to the field of medicine and more particularly to novel combinations and combinatory treatments useful for treating lymphoma. More particularly, the invention thus relates to a combination comprising at least one inhibitor of glutamine metabolism or a pharmaceutically acceptable salt, prodrug, derivative, or sustained release formulation thereof and at least one inhibitor of mitochondrial electron transport chain or a pharmaceutically acceptable salt, prodrug, derivative, or sustained release formulation thereof, for its use in the treatment or the prevention of a lymphoma in a subject in need thereof.

## Description

### FIELD OF THE INVENTION

The invention relates to the field of medicine and more particularly to novel combinations and combinatory treatments useful for treating lymphoma.

### BACKGROUND OF THE INVENTION

Lymphomas, or lymphatic cancers, encompass a family of cancers of the lymphatic system which develops at the expense of lymphocytes. Lymphomas are often described as liquid tumours. They are divided in two main groups which are Hodgkin lymphoma (HL), also known as Hodgkin's disease, and the non-Hodgkin lymphoma (NHL) which comprises around 60 types of diseases. The Hodgkin lymphoma is relatively rare (e.g. HL does not rank in the 20 most common cancers in the UK) and accounts around 10% of all cases of lymphomas. It is characterized by the presence of so-called Reed Sternberg cells which derive from B lymphocytes. HL incidence rate depends on age and is found bimodal: it affects mostly adolescents and young adults in their 20s and adults over 55 (data from https://www.cancer.net/cancer-types/lymphoma-hodgkin/statistics [last accessed on Feb. 13, 2020]), and slightly more male than female. The overall 5-year survival for HL diagnosed is 87%, relapse is observed in around 10% of early stage disease and up to 30% of responders diagnosticated with a late stage disease. Also, around 10% of patients diagnosticated with a late stage disease will be found refractory to chemotherapeutic treatments. Conversely, NHL is a far most common cancer (*e.g*. ranked 6th in the UK) and accounts for 90% of lymphomas and 4% of all new cancer cases in the UK and US in 2016. Unlike HL, incidence of disease increases with age, with a median age for diagnosis of about 71 and 74 in male and female respectively. In western countries, about 90% of people with lymphoma have B-cell NHL. Even though NHL is considered as a cancer responding well to treatments, around 20% of patients do not respond to treatments and around 30% of responders finally experienced relapse after a period of remission. With an incidence rate of 5.0 per 100.000 in 2012, it is estimated a 65% increase of incidence of NHL by 2015. Then there is still a need for treatment of either Hodgkin or non-Hodgkin lymphoma, in particular B cell lymphoma.

Due to their high division rate, cancer cells are in high need of energy. Based on the observation of an abnormal high rate of lactate production, Otto Warburg identified in years 1930s that some cancer cells depend on aerobic glycolysis for energy production, well-known as the Warburg effect. Beside the Warburg effect, mitochondrial phosphorylation (OxPhos) has now been recognized as being another crucial metabolic pathway for energy production. Last years, retrospective analyses have shown that metformin reduces the incidence of certain cancers in diabetic patients. This led to consider metformin as a candidate as a therapy for treating cancer. It is supposed to act by :
i) decreasing circulating glucose insulin production,
ii) inhibiting TCA cycle by inhibiting complex I of electron transport chain and
iii) inhibiting AMPK/mTOR pathways.
Because of its supposed mode of action, it is considered as a promising treatment for solid cancers and reprogrammed chemotherapy cancer cells which became reliant on TCA cycle. Several clinical trials are in course for studying therapeutic effect of metformin used in combination with current chemotherapy and immunotherapy. Chiche et al (2019) have tested an L-asparaginase-temsirolimus based combination therapy in DLBCL patients suffering from a GAPDH low B cell lymphomas, preferentially depending on Oxphos metabolism to produce energy. Treatment was a 4-weeks cycle treatment wherein, in the first two weeks, patient received L-asparaginase and temsirolimus and, in the last two weeks, patient received metformin to sustain therapeutic pressure.

The invention stems in the surprising discovery by the inventors that a combinatory treatment made up of at least one inhibitor of the mitochondrial electron transport chain and at least one inhibitor of glutamine metabolism is particularly effective in killing lymphoma cancer cells, and preferably B lymphoma cancer cells, and this regardless of the metabolic type of the tumour. Furthermore, this treatment has been found to allow an important reduction of tumour size as evaluated by PET-CT scanner resulting in a significant increase of life expectancy in a patient with a B-cell lymphoma refractory to all current therapies. Hence, such a combination is of particular use in treating lymphoma, in particular B cell lymphoma, which represents a significant part of cancers worldwide, and especially refractory disease or relapse lymphoma, in particular B cell lymphoma.

### SUMMARY

Invention thus relates to a combination comprising at least one inhibitor of glutamine metabolism or a pharmaceutically acceptable salt, prodrug, derivative, or sustained release formulation thereof and at least one inhibitor of mitochondrial electron transport chain or a pharmaceutically acceptable salt, prodrug, derivative, or sustained release formulation thereof, for its use in the treatment or the prevention of a lymphoma in a subject in need thereof.

According to a particular embodiment, within said combination of the invention, the at least one inhibitor of glutamine metabolism is selected from telaglenastat, an L-asparaginase, gamma-I-Glutamyl-p-Nitroanilide, (2S)-2-amino-3-phenylmethoxypropanoic acid, 2-phenyl-N-[5-[2-[2-[5-[(2-phenylacetyl)amino]-1,3,4-thiadiazol-2-yl]ethylsulfanyl]ethyl]-1,3,4-thiadiazol-2-yl]acetamide, (2S)-2-amino-6-diazo-5-oxohexanoic acid, 5-[3-bromo-4-(dimethylamino)phenyl]-2,3,5,6-tetrahydro-2,2-dimethyl-benzo[a]phenanthridin-4(1H)-one, 5-(2-Propoxyphenyl)-1H-[1,2,3]triazolo[4,5-d]pyrimidin-7(4H)-one, or 2,2-Dimethyl-beta-alanine or pharmaceutically acceptable salts, prodrugs, derivatives, or sustained release formulations thereof. More particularly, in said combination, the L-asparaginases colaspase or crisantaspase or pharmaceutically acceptable salts, prodrugs, derivatives, or sustained release formulations thereof are particularly preferred, as they display an L-asparaginase and also a glutaminase activity. In an even more particular embodiment, when the at least one inhibitor of glutamine metabolism is an L-asparaginase, said asparaginase or its pharmaceutically acceptable salt, prodrug, derivative, is formulated to be infused every other day from 5000 to 30000 UI/m² in one hour, from 10000 UI/m² to 20000 UI/m² in one hour, preferably 15000UI/m² in one hour, to said subject.

In another particular embodiment, the at least one inhibitor of mitochondrial electron transport chain within the combination of the invention is selected from metformin, phenformin, buformin, (2R,6aS,12aS)-1,2,6,6a,12,12a-hexahydro-2-isopropenyl-8,9-dimethoxychromeno[3,4-b]furo(2,3-h)chromen-6-one, [(1R,2R,6R,10S,11R, 13S,15R,17R)-13-benzyl-6-hydroxy-4,17-dimethyl-5-oxo-15-prop-1-en-2-yl-12,14,18-trioxapentacyclo[11.4.1.01,10.02,6.011,15]octadeca-3,8-dien-8-yl]methyl 2-(4-hydroxy-3-methoxyphenyl)acetate, (E)-N-[(4-hydroxy-3-methoxyphenyl)methyl]-8-methylnon-6-enamide, [(2R,3R)-5,7-dihydroxy-2-(3,4,5-trihydroxyphenyl)-3,4-dihydro-2H-chromen-3-yl] 3,4,5-trihydroxybenzoate, (3R,4S)-3,4-bis(4-hydroxyphenyl)-8-methyl-3,4-dihydro-2H-chromen-7-ol, elesclomol, 6-methyl-N-[(E)-1-pyridin-2-ylethylideneamino]-4a,5-dihydro-2H-[1,2,4]triazino[5,6-b]indol-3-amine or pharmaceutically acceptable salts; prodrugs, derivatives, or sustained release formulations thereof. In a more preferred embodiment, metformin, phenformin or buformin, or their pharmaceutically acceptable salts, prodrugs, derivatives, or sustained release formulations thereof are particularly preferred inhibitor of mitochondrial electron transport chain to be used in the combination of the invention. Indeed, these drugs are currently or have been marketed and therefor their PK/PD data and potential side effect are well known from the skilled in the art, which is of particular advantage. In an even more preferred embodiment metformin, or its pharmaceutically acceptable salts, prodrugs, derivatives, or sustained release formulations thereof, is used in the combination of the invention, because it is known to cause no or few side effects. In another particular embodiment, when the at least one inhibitor of the mitochondrial electron transport chain is a biguanide selected from metformin, buformin or phenformin, or a pharmaceutically acceptable salt, prodrug, derivative thereof, said at least one inhibitor is administered from 0.1 g to 7.5 g daily, preferably from 0.5 g to 1.5 g daily to the subject.

Accordingly, in an embodiment, in the combination of the invention, the at least one inhibitor of glutamine metabolism is a L-asparaginase or a pharmaceutically acceptable salts, prodrugs, derivatives, or sustained release formulations thereof and the at least one inhibitor of mitochondrial electron transport chain is selected from metformin, phenformin or buformin or a pharmaceutically acceptable salts, prodrugs, derivatives, or sustained release formulations thereof.

In said combination of the invention, the compounds (i.e. the at least at least one inhibitor of glutamine metabolism on one side and the at least one inhibitor of mitochondrial electron transport chain) can be formulated for a combined or separate administration. In other words, they can be administered jointly within a single formulation or administered each in a different formulation. Also, when formulated in a different formulation, wherein the compounds can be administered separately at the same time or sequentially.

Though being efficient in effective in killing any type of lymphoma cancer cells, a particular object of the invention is a combination as described above for use in treating a Hodgkin or a Non-Hodgkin lymphoma (NHL). In a preferred embodiment preferably said lymphoma is a B-cell lymphoma, preferentially a B-cell NHL selected from diffuse large B-cell lymphoma (DLBCL), primary effusion lymphoma, mantle cell lymphoma, Burkitt's lymphoma, follicular lymphoma, precursor B-cell lymphoblastic leukemia/lymphoma (B-LBL). in an even more preferred embodiment, the invention relates to a combination as described above for use in treating DLBCL.

In order to avoid the occurrence of unbearable side effects by the subject, it might be desirable to allow a period of rest during which the combination of the invention is not administered to the subject, thereby allowing the organism to recover from such side effects. Accordingly, in another embodiment, the invention, the invention relates to the combination for use in any of the above object or embodiments wherein said combination is administered in cycles, each cycle comprising:
- a first period during which said combination is administered to the subject, and
- a second period of recovery during which said combination is not administered to the subject.

Nevertheless, impeding or slowing as much as possible the progression of the disease during the recovery period, particularly in the case of subject suffering of aggressive disease, is important. Also in an embodiment when the combination of the invention is administered in cycles, at least one mTOR inhibitor is administered to said subject during the recovery period, said mTOR inhibitor being preferably selected from the group consisting in: rapamycin and rapalogs as deferolimus, everolimus, temsirolimus.

Being effective particularly in killing lymphoma cancer cell, the combination of the invention provides a further line of treatment for the above diseases, targeting cells through another pathway or mechanism, which is of particular advantage because of the appearance of resistant cells over time for said treatments. Also, in an embodiment, the combination of the invention combination is administered before and/or after a chemotherapeutic treatment and/or a radiotherapy and/or a graft and/or an immune checkpoint inhibitor treatment. In a particular embodiment, the combination of the invention is administered before or after a chemotherapeutic treatment based on a regimen selected from CHOP, R-CHOP or R-EPOCH, R-ACVBP, for treating DLBCL.

Also, being a novel treatment of lymphoma, the combination of the invention is of particular interest in treating subject which are found relapse or refractory to current or other treatments. Also, in a particular embodiment, the invention relates to the combination, combination for its use as exposed above, in a subject who is relapse or refractory to chemotherapy and/or graft and/or checkpoint inhibitor therapy and/or radiotherapy.

Combinatory treatment made up of at least one inhibitor of the mitochondrial electron transport chain and at least one inhibitor of glutamine metabolism is found particularly effective in killing lymphoma cancer cell of any metabolic subtype. Accordingly, in an embodiment, the invention relates to said combination as exposed above for its use in treating said lymphoma wherein said combination induces cytotoxicity in glycolytic and OXPHOS type cancer cells from lymphoma.

In a more particular embodiment, the invention relates to the combination as described above which prevents resistance of cancer cells to metformin.

Further advantages and features of the invention are exposed in the following description hereby provided in an illustrative and a non-limitative way.

### FIGURE LEGENDS

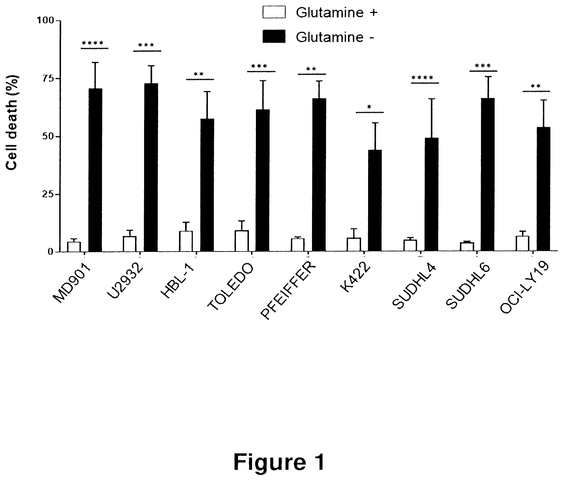

Figure 1: DLBCL cells critically rely on Glutamine for their survival, independently of their COO or CCC classification. Glutamine deprivation induces massive cell death of DLBCL cells. A panel of 9 DLBCL cell lines of different CCC or COO subtypes were grown either with or without 2 mM glutamine for 72 hours, and cell viability was evaluated by Trypan blue staining. Student's t-test, * p<0.05; ** p<0.01; ***p<0.001 ****p<0.0001.

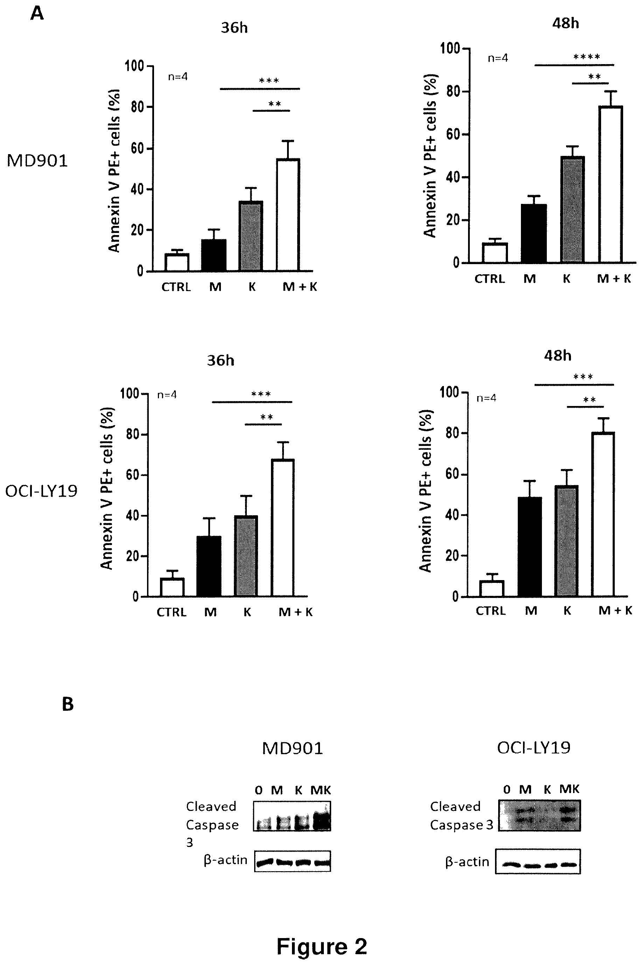

**Figure 2****: Combination of the invention induces massive apoptosis in DLBCL cell lines.** (A) MD901 and OciLy19 cell lines were both treated with metformin 2.5mM (M) either alone or in combination with 2UI/mL of L-asparaginase (Kidrolase, K) for the indicated time and were monitored for apoptosis by AnnexinV-PE staining followed by FACS analysis. (B) Combination of metformin and L-asparaginase induces increased caspase 3 cleavage in DLBCL cells. Whole cell extracts from MD901 (COO subtype : ABC) and Ocily19 (COO subtype : GC) were treated as in (A) and were monitored for apoptosis by immunoblotting for cleaved caspase 3. Means +/- SEM of three independent experiments for each DLBCL cell line is reported. Student's t-test, * p<0.05; ** p<0.01; **** p<0.0001.

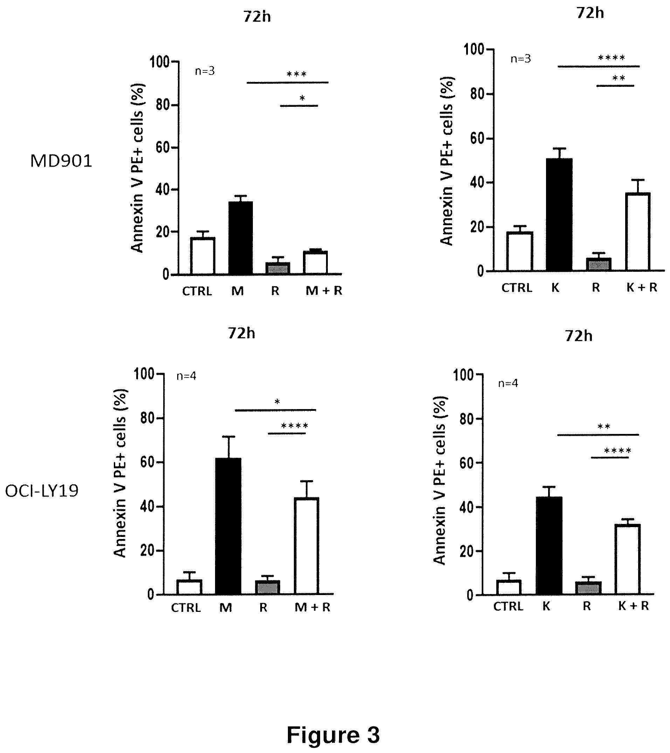

**Figure 3****: Rapamycin antagonizes metformin and L-asparaginase pro-apoptotic effect in DLBCL cell lines.** MD901 and OciLy19 DLBCL cell lines were treated with rapamycin 20 nM (R) along with either metformin 2.5mM (M) or 2 UI/mL of Kidrolase (K) and monitored for apoptosis by AnnexinV-PE staining followed by FACS analysis. Means +/- SEM of at least three independent experiments for each DLBCL cell line is reported. Student's t-test,* p<0.05; ** p<0.01; **** p<0.0001.

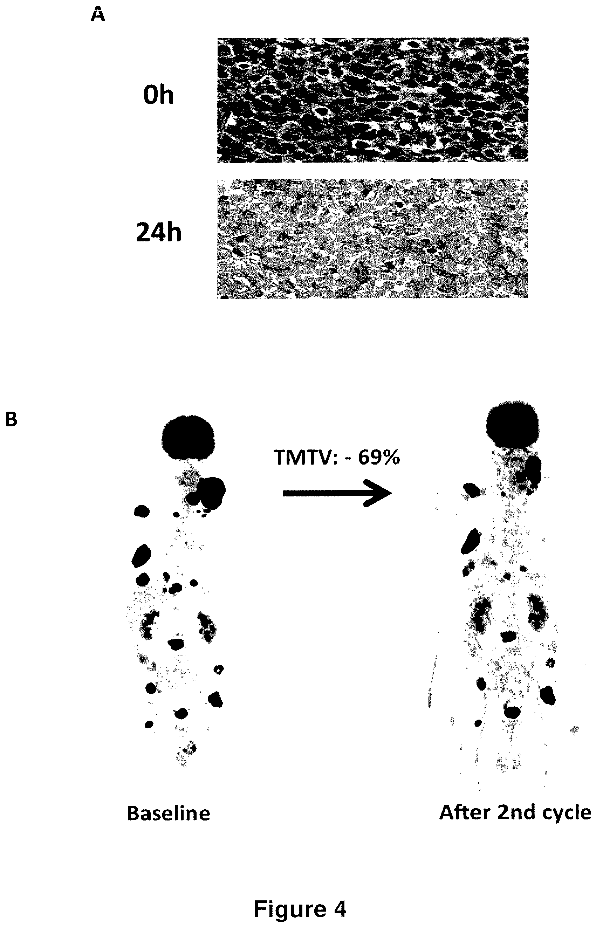

**Figure 4****: metformin and L-asparaginase combinatory treatment induces progressive remission in a relapsed/refractory DLBCL patient.** (A) Combinatory treatment of metformin and L-asparaginase induces a massive necrosis in a lymph node within few hours upon administration as observed between baseline (0h) and 24 hours post-administration on a biopsy from lymph nodes HE coloured and observed under optical microscopy (400x). (B) ⁸F-FDG-PET-CT imaging at baseline e.g. before cycle 1 of combinatory treatment with metformin and L-asparaginase compared to ¹⁸F-FDG-PET-CT imaging realized after cycle 2 which shows a marked decrease of cancer expansion. Said marked decrease of TMTV (total metabolic tumor volume) representing a 69% reduction of the tumor volume.

### DETAILED DESCRIPTION AND ADDITIONAL EMBODIMENTS

Inventors have discovered that B cell lymphoma are particularly sensitive to glutamine starvation, regardless of the subtype, notably their metabolic dependency, they belong to. Moreover, they surprisingly found that the combined use of an inhibitor of glutamine metabolism along with an inhibitor of the mitochondrial electron transport chain results in increased cell death by apoptosis. Effectiveness of combinatory treatments as disclosed herein has been confirmed *in vivo* in relapse refractory patient to whom therapeutic solution is no longer available, making combinatory treatment of the invention particularly valuable for patients facing therapeutic dead-end.

Hence, the subject-matter of the present invention provides new combinatory treatments suitable for treating Hodgkin's or non-Hodgkin lymphomas and in particular to non-Hodgkin B cell lymphoma which refers to diseases such as diffuse large B-cell lymphoma (DLBCL), primary effusion lymphoma, mantle cell lymphoma, Burkitt's lymphoma, follicular lymphoma, precursor B-cell lymphoblastic leukemia/lymphoma (B-LBL), preferably to DLBCL.

### Definitions

As used herein, "treatment" or "to treat" include the therapy, the prevention, the prophylaxis, the retardation or the reduction of symptoms provoked by or of the causes of a disease. When related to lymphoma more particularly includes stopping, stabilizing or slowing down the progression of a disease, or lessening the severity of its symptoms. In other words, it encompasses the eradication of a tumour, decreasing the size of a tumour, stopping or slowing down the development of tumour. More, particularly it encompasses the eradication, reduction in size, stopping or slowing down of the growth and invasion of lymph nodes and/or extranodal masses (i.e. clumps of cancerous cells gathering in the body somewhere else than in lymph nodes. The terms "treatment" or "to treat" also encompasses therapy, prevention, prophylaxis, retardation or reduction of symptoms of the disease in a subject who has been subjected to a treatment for this lymphoma and who, after a remission period, is experiencing or suspected to experience relapse. Also, "treatment" encompasses therapy, prevention, prophylaxis, retardation or reduction of symptoms of lymphomas refractory to current treatments as chemotherapeutic treatment.
The terms "subject" or "subject in need thereof" as used herein refer to a mammal, preferably a human, who is suffering or suspected to suffer from a lymphoma, in particular a B cell lymphoma. In a particular embodiment, said subject experiences or is suspected to experience relapse, meaning that after a period of complete remission following *e.g.,* a chemotherapeutic treatment, a radiotherapy, a CAR T-cell therapy, immune therapy with *e.g.* immune check point inhibitors or a graft, lymphoma, in particular a B cell lymphoma, is found to return in said patient. In another embodiment, said subject has previously been subjected to a treatment for its lymphoma and is experiencing stabilization, an improvement (*e.g*. a decrease in size of at least one primary tumour and/or secondary tumour and/or metastasis), or a partial response to said treatment. In a further embodiment said subject is found refractory to the treatment, meaning that the disease is getting worse for said subject despite current treatment (*e.g*. chemotherapeutic treatment, immunotherapy with for example immune check point inhibitors, graft, CAR T-cell therapy).
The terms "Combination" or "combinatorial treatment/therapy" or "combinatory treatment" as used herein designate a treatment of a disease or disorder wherein at least two or more drugs and/or active compounds are co-administered to a subject to cause a biological effect. In a combined therapy according to this invention, the drugs may be administered at the same time, together or separately, or sequentially. Also, they may be administered through different routes and protocols. For example, one of the at least two or more drugs can be formulated to be administered orally and one of others can be administered intravenously. Although, the at least two or more drugs and/or active compounds might be formulated together, the at least two or more drugs and/or active compounds might also be formulated separately.
As used herein, the term "compound" or "drug" designates the chemical compound or drug as named in the application, as well as any pharmaceutically acceptable salt, hydrate, stereoisomer, racemate, conjugate, pro-drug thereof, of any purity.
The term "salt" or "salts" refers to a pharmaceutically acceptable and relatively non-toxic, inorganic or organic acid addition salt of a compound of the present invention. Salt selection is now a common standard operation in the process of drug development. Though, most salts of a given active principle are bioequivalents, some may have, among others, increased solubility or bioavailability properties. Regarding compounds which have already been developed and/or approved as a medication, preferred salts for these compounds will be those as already developed and/or approved.
The term "prodrug" refers to a precursor of a compound which upon administration, generates said compound upon chemical or enzymatic reaction. Prodrug design is a common standard operation in the process of drug development. Regarding compounds which have already been developed and/or approved as a medication, preferred prodrugs for these compounds, if any, will be those as already developed and/or approved.

### Combinations of the invention

Lymphoma cell lines were surprisingly found particularly sensitive to glutamine starvation, regardless of their so-called metabolic subtype.

Without being bound to any theory, L-asparaginase is known to display at least the following activities:
- an asparaginase activity by catalysing the conversion of L-asparagine into aspartic acid and ammonia, and
- a glutaminase activity by catalysing the conversion of L-glutamine into L-glutamate and ammonia, resulting in the degradation of extracellular glutamine and thus glutamine starvation of the cells.

Furthermore, metformin is known for inducing cell death in some solid cancers in a pleiotropic way.

Inventors further surprisingly found that a combination of L-asparaginase along with metformin results in a potentiated cytotoxic effect on lymphoma cells compared to what is seen with each drug alone.

The present invention therefore relates to a combination comprising:
- at least one inhibitor of glutamine metabolism or a salt, pro-drug, derivative, or sustained release formulation thereof and
- at least one mitochondrial electron transport chain or a salt, prodrug, derivative, or sustained release formulation thereof.

Examples of such compounds are listed, without being limited to, in table 1 below.

**Table 1**

| Compound (INN/IUPAC name) | CAS number(s) |
|---|---|
| Glutamine metabolism inhibitors | |
| telaglenastat | 1439399-58-2 |
| colaspase | 9015-68-3 |
| crisantaspase | 1349719-22-7 |
| gamma-l-Glutamyl-p-Nitroanilide | 67953-08-6 |
| | 7300-59-6 |
| (2S)-2-amino-3-phenylmethoxypropanoic acid | 4726-96-9 |
| 2-phenyl-N-[5-[2-[2-[5-[(2-phenylacetyl)amino]-1,3,4-thiadiazol-2-yl]ethylsulfanyl]ethyl]-1,3,4-thiadiazol-2-yl]acetamide | 314045-39-1 |
| (2S)-2-amino-6-diazo-5-oxohexanoic acid | 157-03-9 |
| 5-[3-bromo-4-(dimethylamino)phenyl]-2,3,5,6-tetrahydro-2,2-dimethyl-benzo[a]phenanthridin-4(1H)-one | 311795-38-7 |
| 5-(2-Propoxyphenyl)-1H-[1,2,3]triazolo[4,5-d]pyrimidin-7(4H)-one | 37762-06-4 |

| 2,2-Dimethyl-beta-alanine | 19036-43-2 |
|---|---|
| Inhibitors of mitochondrial electron transport chain | |
| (2*R*,6aS,12a*S*)-1,2,6,6a,12,12a-hexahydro-2-isopropenyl-8,9-dimethoxychromeno[3,4-*b*]furo(2,3-h)chromen-6-one | 83-79-4 |
| (known as rotenone) | |
| [(1R,2R,6R, 10S, 11R,13S,15R,17R)-13-benzyl-6-hydroxy-4,17-dimethyl-5-oxo-15-prop-1 -en-2-yl-12,14,18-trioxapentacycto[11.4.1.01,10.02,6.011,15]octadeca-3,8-dien-8-yl]methyl 2-(4-hydroxy-3-methoxyphenyl)acetate | 57444-62-9 |
| (known as resiniferatoxin) | |
| (*E*)-*N*-[(4-hydroxy-3-methoxyphenyl)methyl]-8-methylnon-6-enamide | 404-86-4 |
| (known as capsaicin) | |
| [(2*R*,3*R*)-5,7-dihydroxy-2-(3,4,5-trihydroxyphenyl)-3,4-dihydro-2*H-*chromen-3-yl] 3,4,5-trihydroxybenzoate (known as epigallocatechin-3 gallate) | 989-51-5 |
| (3*R*,4*S*)-3,4-bis(4-hydroxyphenyl)-8-methyl-3,4-dihydro-2*H-*chromen-7-ol | 1374524-68-1 |
| metformin | 657-24-9 |
| | 1115-70-4 |
| buformin | 692-13-7 |
| phenformin | 114-86-3 |
| elesclomol | 488832-69-5 |
| 6-methyl-N-[(E)-1-pyridin-2-ylethylideneamino]-4a,5-dihydro-2H-[1,2,4]triazino[5,6-b]indol-3-amine | 1622945-04-3 |

Inhibitors of glutamine metabolism suitable for the combinations of the invention should be able to induce glutamine starvation in cancer cells, which can be easily tested *in vitro* using well-known methods from the skilled in the art and commercially available kits. Inhibitors of glutamine metabolism can be selected from glutaminase inhibitors e.g. telaglenastat, (2S)-2-amino-6-diazo-5-oxohexanoic acid, 2-phenyl-N-[5-[2-[2-[5-[(2-phenylacetyl)amino]-1,3,4-thiadiazol-2-yl]ethylsulfanyl]ethyl]-1,3,4-thiadiazol-2-yl]acetamide, 5-(2-Propoxyphenyt)-1H-[1,2,3]triazoto[4,5-d]pyrimidin-7(4H)-one or 5-[3-bromo-4-(dimethylamino)phenyl]-2,3,5,6-tetrahydro-2,2-dimethyl-benzo[a]phenanthridin-4(1H)-one, from blockers of ASCT2 or SNAT2 glutamine transporter *e.g.* (2S)-2-amino-3-phenylmethoxypropanoic acid, gamma-l-Glutamyl-p-Nitroanilide, 2,2-Dimethyl-beta-alanine, or from L-asparaginases, *e.g.* L-asparaginase derived from *Escherichia coli* (colaspase, CAS number 9015-68-3) or PEGylated or non-PEGylated L-Asparaginase from *Erwinia chrysanthemi* (crisantaspase, CAS number 1349719-22-7) providing that said L-asparaginase has retained its glutaminase activity. Although mutant asparaginases that do not exhibit asparaginase activity exists in the art, L-asparaginase are particularly preferred when displaying both asparaginase and glutaminase activities.

Mitochondrial electron transport chain inhibitors can be selected from inhibitors of one or more of respiratory complexes (*i.e.* inhibitor of complex I, II, III and/or IV) or of cytochrome C, such asrotenoids *e.g.* rotenone, , vanilloids *e.g.* resiniferatoxin or capsaicin, alkaloids *e.g*., biguanides *e.g.* metformin, buformin, or phenformin, polyphenols *e.g.* epigallocatechin-3 gallate or (3*R*,4*S*)-3,4-bis(4-hydroxyphenyl)-8-methyl-3,4-dihydro-2*H*-chromen-7-ol and generalized inhibitors of ETC activity *e.g.* elesclomol or 6-methyl-*N*-[(*E*)-1-pyridin-2-ylethylideneamino]-4a,5-dihydro-2*H* [1,2,4]triazino[5,6-b]indol-3-amine), or combinations thereof.

An embodiment of the present invention relates to a combination comprising at least:
- an inhibitor of glutamine metabolism selected from telaglenastat; colaspase; crisantaspase; gamma-l-Glutamyl-p-Nitroanilide; (2S)-2-amino-3-phenylmethoxypropanoic acid; 2-phenyl-N-[5-[2-[2-[5-[(2-phenylacetyl)amino]-1,3,4-thiadiazol-2-yl]ethylsulfanyl]ethyl]-1,3,4-thiadiazol-2-yl]acetamide; (2S)-2-amino-6-diazo-5-oxohexanoic acid; 5-[3-bromo-4-(dimethylamino)phenyl]-2,3,5,6-tetrahydro-2,2-dimethyl-benzo[a]phenanthridin-4(1H)-one; 5-(2-Propoxyphenyl)-1H-[1,2,3]triazolo[4,5-d]pyrimidin-7(4H)-one; or 2,2-Dimethyl-beta-alanine, a pharmaceutically acceptable salt, prodrug, derivative, or sustained release formulation thereof, and
- at least one inhibitor of mitochondrial electron transport chain selected from metformin; phenformin; buformin;; (2R,6aS,12aS)-1,2,6,6a,12,12a-hexahydro-2-isopropenyl-8,9-dimethoxychromeno[3,4-b]furo(2,3-h)chromen-6-one; [(1R,2R,6R,10S,11R,13S,15R,17R)-13-benzyl-6-hydroxy-4,17-dimethyl-5-oxo-15-prop-1-en-2-yl-12,14,18-trioxapentacyclo[11.4.1.01,10.02,6.011,15]octadeca-3,8-dien-8-yl]methyl 2-(4-hydroxy-3-methoxyphenyl)acetate; (E)-N-[(4-hydroxy-3-methoxyphenyl)methyl]-8-methylnon-6-enamide; [(2*R*,3*R*)-5,7-dihydroxy-2-(3,4,5-trihydroxyphenyl)-3,4-dihydro-2*H*-chromen-3-yl] 3,4,5-trihydroxybenzoate; (3*R*,4*S*)-3,4-bis(4-hydroxyphenyl)-8-methyl-3,4-dihydro-2H-chromen-7-ol;Elesclomol; 6-methyl-N-[(E)-1-pyridin-2-ylethylideneamino]-4a,5-dihydro-2H-[1,2,4]triazino[5,6-b]indol-3-amine or a pharmaceutically acceptable salt, prodrug, derivative, or sustained release formulation thereof.

Biguanides are drugs well-known in the art. Biguanides are known to have an inhibitory activity of complex I of mitochondrial electron transport chain in cells. The skilled in the art know how to detect such inhibitory activity, e.g. *in vitro* on cultured cells. A biguanide suitable for its use in the combination according to the invention can be selected from the list: metformin, phenformin or buformin. Preferred biguanides are metformin or phenformin, metformin being more particularly preferred as being known in the art as safe and currently approved. These drugs indeed lower gluconeogenesis which might be of further interest in treating cancer.

In a particular embodiment of the invention, said at least one mitochondrial electron transport chain inhibitor is a biguanide. In a more particular embodiment said biguanide is selected from metformin, phenformin or buforminor a pharmaceutically acceptable salt, prodrug, derivative, or sustained release formulation thereof. Therefore, in a more particular embodiment said combination comprises, at least one glutamine metabolism inhibitor listed in table 1 or a salt, pro-drug, derivative, or sustained release formulation thereof, and a biguanide selected from metformin, phenformin or buformin, or a pharmaceutically acceptable salt, prodrug, derivative, or sustained release formulation thereof. In an even more particular embodiment said combination comprises at least one inhibitor of glutamine metabolism listed in table 1 or a salt, pro-drug, derivative, or sustained release formulation thereof, and a biguanide selected from metformin, phenformin or buformin or a pharmaceutically acceptable salt, prodrug, derivative, or sustained release formulation thereof. In a particular embodiment said combination comprises at least one inhibitor of glutamine metabolism listed in table 1 or a salt, pro-drug, derivative, or sustained release formulation thereof, and metformin a pharmaceutically acceptable salt, prodrug, derivative, or sustained release formulation thereof. In another particular embodiment, combination according to the invention comprises at least one inhibitor of glutamine metabolism listed in table 1 or a salt, pro-drug, derivative, or sustained release formulation thereof, and phenformin or a pharmaceutically acceptable salt, prodrug, derivative, or sustained release formulation thereof. In another particular embodiment, combination according to the invention comprises at least one inhibitor of glutamine metabolism listed in table 1 or a salt, pro-drug, derivative, or sustained release formulation thereof, and buformin or a pharmaceutically acceptable salt, prodrug, derivative, or sustained release formulation thereof. In another particular embodiment, combination according to the invention comprises at least one inhibitor of glutamine metabolism listed in table 1 or a salt, pro-drug, derivative, or sustained release formulation thereof, and elesclomol or a pharmaceutically acceptable salt, prodrug, derivative, or sustained release formulation thereof. In another particular embodiment, combination according to the invention comprises at least one inhibitor of glutamine metabolism listed in table 1 or a salt, pro-drug, derivative, or sustained release formulation thereof, and 6-methyl-N-[(E)-1-pyridin-2-ylethylideneamino]-4a,5-dihydro-2H-[1,2,4]triazino[5,6-b]indol-3-amine or a pharmaceutically acceptable salt, prodrug, derivative, or sustained release formulation thereof. In another particular embodiment, combination according to the invention comprises at least one inhibitor of glutamine metabolism listed in table 1 or a salt, pro-drug, derivative, or sustained release formulation thereof, and epigallocatechin-3 gallate or a pharmaceutically acceptable salt, prodrug, derivative, or sustained release formulation thereof. In another particular embodiment, combination according to the invention comprises at least one inhibitor of glutamine metabolism listed in table 1 or a salt, pro-drug, derivative, or sustained release formulation thereof, and (3*R*,4*S*)-3,4-bis(4-hydroxyphenyl)-8-methyl-3,4-dihydro-2*H*-chromen-7-ol or a pharmaceutically acceptable salt, prodrug, derivative, or sustained release formulation thereof. In another particular embodiment, combination according to the invention comprises at least one inhibitor of glutamine metabolism listed in table 1 or a salt, pro-drug, derivative, or sustained release formulation thereof, and capsaicin or a pharmaceutically acceptable salt, prodrug, derivative, or sustained release formulation thereof. In another particular embodiment, combination according to the invention comprises at least one inhibitor of glutamine metabolism listed in table 1 or a salt, pro-drug, derivative, or sustained release formulation thereof, and resiniferatoxin or a pharmaceutically acceptable salt, prodrug, derivative, or sustained release formulation thereof. In another particular embodiment, combination according to the invention comprises at least one inhibitor of glutamine metabolism listed in table 1 or a salt, pro-drug, derivative, or sustained release formulation thereof, and rotenone or a pharmaceutically acceptable salt, prodrug, derivative, or sustained release formulation thereof.

In a particular embodiment of the invention, said at least one inhibitor of glutamine metabolism is at least one of inhibitors glutamine metabolism listed in table 1. In a more particular embodiment said at least one of inhibitor of glutamine metabolism is a L-asparaginase is selected from colaspase or PEGylated or non-PEGylated crisantaspase, a pharmaceutically acceptable salt, prodrug, derivative, or sustained release formulation thereof. Therefore, in an embodiment, invention relates to a combination comprising at least a L-asparaginase selected from colaspase or PEGylated or non-PEGylated crisantaspase, a pharmaceutically acceptable salt, prodrug, derivative, or sustained release formulation thereof, and at least one inhibitor of mitochondrial electron transport chain. In another embodiment, the invention relates to at least a L-asparaginase selected from colaspase or PEGylated or non-PEGylated crisantaspase, a pharmaceutically acceptable salt, prodrug, derivative, or sustained release formulation thereof, and at least one inhibitor of mitochondrial electron transport chain listed in table 1. In a particular embodiment, the invention relates to combination comprising at least colaspase or a pharmaceutically acceptable salt, prodrug, derivative, or sustained release formulation thereof, and at least one inhibitor of mitochondrial electron transport chain listed in table 1. In another particular embodiment, the invention relates to combination comprising at least PEGylated or non-PEGylated crisantaspase or a pharmaceutically acceptable salt, prodrug, derivative, or sustained release formulation thereof, and at least one inhibitor of mitochondrial electron transport chain listed in table 1. In a particular embodiment, the invention relates to combination comprising at least gamma-l-Glutamyl-p-Nitroanilide or a pharmaceutically acceptable salt, prodrug, derivative, or sustained release formulation thereof, and at least one inhibitor of mitochondrial electron transport chain listed in table 1. In a particular embodiment, the invention relates to combination comprising at least (2S)-2-amino-3-phenylmethoxypropanoic acid or a pharmaceutically acceptable salt, prodrug, derivative, or sustained release formulation thereof, and at least one inhibitor of mitochondrial electron transport chain listed in table 1. In a particular embodiment, the invention relates to combination comprising at least 2-phenyl-N-[5-[2-[2-[5-[(2-phenylacetyl)amino]-1,3,4-thiadiazol-2-yl]ethylsulfanyl]ethyl]-1,3,4-thiadiazol-2-yl]acetamide or a pharmaceutically acceptable salt, prodrug, derivative, or sustained release formulation thereof, and at least one inhibitor of mitochondrial electron transport chain listed in table 1. In a particular embodiment, the invention relates to combination comprising at least (2S)-2-amino-6-diazo-5-oxohexanoic acid or a pharmaceutically acceptable salt, prodrug, derivative, or sustained release formulation thereof, and at least one inhibitor of mitochondrial electron transport chain listed in table 1. In a particular embodiment, the invention relates to combination comprising at least 5-[3-bromo-4-(dimethylamino)phenyl]-2,3,5,6-tetrahydro-2,2-dimethyl-benzo[a]phenanthridin-4(1H)-one or a pharmaceutically acceptable salt, prodrug, derivative, or sustained release formulation thereof, and at least one inhibitor of mitochondrial electron transport chain listed in table 1.

In a particular embodiment, the invention relates to a combination comprising at least a L-asparaginase selected from colaspase or PEGylated or non-PEGylated crisantaspase, a pharmaceutically acceptable salt, prodrug, derivative, or sustained release formulation thereof, and at least one inhibitor of mitochondrial electron transport chain selected from metformin, phenformin or buformin or a pharmaceutically acceptable salt, prodrug, derivative, or sustained release formulation thereof.

In a particular embodiment, the invention relates to a combination comprising at least a L-asparaginase selected from colaspase or PEGylated or non-PEGylated crisantaspase, a pharmaceutically acceptable salt, prodrug, derivative, or sustained release formulation thereof, at least one biguanide selected from metformin, phenformin or buformin or a pharmaceutically acceptable salt, prodrug, derivative, or sustained release formulation thereof and at least one other one inhibitor of mitochondrial electron transport chain listed in table 1.

In a further particular embodiment, said at least one glutamine metabolism inhibitor, within said combination, is colaspase or a pharmaceutically acceptable salt, prodrug, derivative, or sustained release formulation thereof. In a more particular embodiment said combination comprises at least one inhibitor of the mitochondrial electron transport chain and/or at least one biguanide preferably selected from metformin, phenformin or buforminor a pharmaceutically acceptable salt, prodrug, derivative, or sustained release formulation thereof, and colaspase or a pharmaceutically acceptable salt, prodrug, derivative, or sustained release formulation thereof.

In another particular embodiment, said at least one glutamine metabolism inhibitor, within said combination, is crisantaspase or a pharmaceutically acceptable salt, prodrug, derivative, or sustained release formulation thereof. In a more particular embodiment said combination comprises at least one inhibitor of the mitochondrial electron transport chain and/or at least one biguanide preferably selected from metformin, phenformin or buformin or a pharmaceutically acceptable salt, prodrug, derivative, or sustained release formulation thereof, and crisantaspase or a pharmaceutically acceptable salt, prodrug, derivative, or sustained release formulation thereof.

In a very particular embodiment, the invention relates to a combination comprising colaspase or a pharmaceutically acceptable salt, prodrug, derivative, or sustained release formulation thereof and metformin a pharmaceutically acceptable salt, prodrug, derivative, or sustained release formulation thereof.

As it will be detailed below, in a combination therapy according to the present invention the compounds or drugs can be formulated separately or together. When administered separately they can be administered concomitantly or sequentially. Administration scheme can vary depending on PK/PD specification of each administered compound, or of compound in a given formulation. In a preferred embodiment, administration the combinatory treatment of the invention is designed in such a way the compounds, their active moiety or active derivative(s), act together to provide the fulness of efficiency of the combination, once delivered to the subject.

Furthermore, combinations of the invention thought to be particularly effective comprise at least 1, 2, 3, 4, or even more drugs or compounds or active agents in addition to the combinatory treatment according to the invention. Surprisingly, it has been observed that mTOR inhibitors antagonize the effect of drugs of the combinatory treatment of the invention. Thus, combination treatment of the invention should not comprise mTOR inhibitor in such a way that it could antagonize the effect of compound of combination of the invention. However, mTOR inhibitors could be used separately, *e.g.,* between cycle or during recovery period as a consolidating phase, in order to stabilize the disease while combination is no more administered.

### Therapeutic methods and uses of the compounds of the invention

Since inventors of the present invention found that lymphoma cell lines, in particular B cells lymphoma, are particularly sensitive to glutamine deprivation, regardless of their dependency on oxidative phosphorylation (OXPHOS) or glycolysis, or their COO subtype. Thus, a broader and ubiquitous spectrum of action than current lymphoma treatments is to be expected. This is even more of particular interest when considering the heterogeneity of cancer cell types that may exist within a single tumour.

Further, a particularly strong induction of apoptosis and cell death is observed upon exposure of lymphoma cell lines, in particular B cells lymphoma, to the combinatory treatment of the invention which comprises at least one inhibitor of glutamine metabolism and at least one inhibitor of the mitochondrial electron transport chain leading to potentiation of cytotoxicity of the compounds on the lymphoma cell lines. Cytotoxicity is particularly potentiated on B cell lymphomas. An actual clinical benefit provided to a relapse and refractory subject suffering from DLBCL results from the administration of a combination of the invention.

Accordingly, an object of the invention relates to a combination comprising at least one inhibitor of glutamine metabolism or a salt, prodrug, derivative, or sustained release formulation thereof and at least one inhibitor of the mitochondrial electron transport chain or a salt, prodrug, derivative, or sustained release formulation thereof, for its use in the treatment or the prevention of a lymphoma, more particularly B cell lymphoma, in a subject in need thereof.

B cell lymphoma are type of lymphoma that affect B cells. B cell lymphoma are both Hodgkin or non-Hodgkin lymphoma. Non-Hodgkin B cell lymphoma encompasses diffuse large B-cell lymphoma (DLBCL), primary effusion lymphoma, mantle cell lymphoma, Burkitt's lymphoma, follicular lymphoma, Precursor B-cell lymphoblastic leukaemia/lymphoma (B-LBL). Also a particular embodiment of the invention relates to a combination comprising at least one inhibitor of glutamine metabolism or a salt, prodrug, derivative, or sustained release formulation thereof and at least one inhibitor of the mitochondrial electron transport chain or a pharmaceutically acceptable salt, prodrug, derivative, or sustained release formulation thereof, for its use in the treatment or the prevention of B cell lymphoma are type of lymphoma that affect B cells. B cell lymphoma are both Hodgkin or non-Hodgkin lymphoma. Non-Hodgkin B cell lymphoma encompasses diffuse large B-cell lymphoma (DLBCL), primary effusion lymphoma, mantle cell lymphoma, Burkitt's lymphoma, follicular lymphoma, Precursor B-cell lymphoblastic leukaemia/lymphoma (B-LBL).

Even if combination of the invention is suitable for any of the above disease, it is particularly efficient in a DLBCL suffering subjects. Also, an even more particular object of the invention relates to a combination comprising at least one least one inhibitor of glutamine metabolism a pharmaceutically acceptable salt, prodrug, derivative, or sustained release formulation thereof and at least one inhibitor of the mitochondrial electron transport chain or a pharmaceutically acceptable salt, prodrug, derivative, or sustained release formulation thereof, for its use in the treatment or the prevention of DLBCL, in a subject in need thereof.

In a particular embodiment, in the combinatory therapies of the invention, the at least one inhibitor of glutamine metabolism and the at least one inhibitor of the mitochondrial electron transport chain are chosen among those which are already developed, and for which data (side effects, PK/PD, etc) are already available to the skilled in the art. Such inhibitors of glutamine metabolism can be selected from, for example, glutaminase inhibitors (*e.g*. telaglenastat), blockers of ASCT2 glutamine transporter (*e.g*. gamma-l-Glutamyl-p-Nitroanilide or 2,2-Dimethyl-beta-alanine), or from L-asparaginases (*e.g.* colaspase), (*e.g.* pegylated) or not pegylated crisantaspase.

In a preferred embodiment, said inhibitors of glutamine metabolism is L-asparaginases because they display, both glutaminase and asparaginase activities which is also of particular interest when considering treating cancer. L-asparaginases are often used in treating tumours expressing high levels of asparagine synthetase. IBecause of its glutaminase activity, L-asparaginase is of interest for its use for all type of B lymphoma cell lines, more particularly DLBCL cell lines, as cell lymphoma are all sensitive to glutamine deprivation. Also, biguanide well-known in the art are metformin, phenformin or buformin which have been or are currently on the market for several therapeutic indications.

Accordingly, the present invention relates to a combination comprising at least:
- an inhibitor of glutamine metabolism selected from telaglenastat, colaspase, crisantaspase or a pharmaceutically acceptable salt, prodrug, derivative, or sustained release formulation thereof, and
- an inhibitor of the mitochondrial electron transport chain selected from metformin, phenformin or buformin or a pharmaceutically acceptable salt, prodrug, derivative, or sustained release formulation thereof,
for its use in the treatment or the prevention of a lymphoma in a subject in need thereof.

In a more particular embodiment, the invention relates to said combination for its use for treating Hodgkin or non-Hodgkin lymphoma in a subject in need thereof. In an even more particular embodiment said Non-Hodgkin lymphoma is a B cell lymphoma is selected from diffuse large B-cell lymphoma (DLBCL), primary effusion lymphoma, mantle cell lymphoma, Burkitt's lymphoma, follicular lymphoma, Precursor B-cell lymphoblastic leukemia/lymphoma (B-LBL). A particularly preferred object of the invention relates to said combination for its use for treating DLBCL.

Though certain biguanides developed as a medicine (*e.g*., phenformin, metformin or buformin) have been shown to disrupt the Warburg effect, metformin is the one reported to have no or few side effects. Also, in another embodiment, the invention relates to a combination comprising metformin or a pharmaceutically acceptable salt, prodrug, derivative, or sustained release formulation thereof and at least one glutamine metabolism selected from telaglenastat, a L-asparaginase, (*e.g*. colaspase or crisantaspase), or a pharmaceutically acceptable salt, prodrug, derivative, or sustained release formulation thereof, for its use for treating lymphoma in a subject in need thereof.

As mentioned above, all biguanide known to be if use in lowering blood glucose (e.g. phenformin and metformin) might be considered of particular interest in the frame of cancer treatment. Also in another embodiment, the invention relates to a combination comprising phenformin or a pharmaceutically acceptable salt, prodrug, derivative, or sustained release formulation thereof and at least one glutamine metabolism selected from telaglenastat, a L-asparaginase, (*e.g*. colaspase or crisantaspase), or a pharmaceutically acceptable salt, prodrug, derivative, or sustained release formulation thereof, for its use for treating lymphoma in a subject in need thereof.

In another embodiment the invention relates to the invention relates to a combination comprising a biguanide selected from metformin, phenformin, buformin, or proguanil and another inhibitor of mitochondrial electron transport chain as listed in table 1 and at least one glutamine metabolism selected from telaglenastat, a L-asparaginase, (*e.g*. colaspase or crisantaspase), or a pharmaceutically acceptable salt, prodrug, derivative, or sustained release formulation thereof, for its use for treating lymphoma in a subject in need thereof.

In a more particular embodiment said subject is suffering from a B-cell lymphoma, preferably Hodgkin or a non-Hodgkin B cell lymphoma, more preferably said subject is suffering from a B cell NHL, even more preferably from a B cell lymphoma selected from DLBCL, primary effusion lymphoma, mantle cell lymphoma, Burkitt's lymphoma, follicular lymphoma, B-LBL. In a preferred embodiment, said subject is suffering or suspected to suffer from DLBCL.

As explained above, L-asparaginases are particularly preferred glutamine metabolism inhibitors because of their dual glutaminase and asparaginase activities that might be of some use in the frame of cancer treatment. Accordingly, in a particular embodiment, the invention relates to a combination comprising at least one L-asparaginase, and at least one biguanide preferably selected from metformin, phenformin or buformin or a pharmaceutically acceptable salt, prodrug, derivative, or sustained release formulation thereof, for its use for treating lymphoma in a subject in need thereof. In another particular embodiment said subject is suffering from a B cell lymphoma. In another particular embodiment said subject is suffering from a Hodgkin or a non-Hodgkin B cell lymphoma. In another particular embodiment said subject is suffering from a B cell NHL. In another particular embodiment said subject is suffering from a B cell NHL selected from DLBCL, primary effusion lymphoma, mantle cell lymphoma, Burkitt's lymphoma, follicular lymphoma, B-LBL, preferably the B cell NHL is DLBCL.

In a particular embodiment, the invention relates to a combination comprising metformin and an L-asparaginase selected from colaspase or crisantaspase or a pharmaceutically acceptable salt, prodrug, derivative, or sustained release formulation thereof, for its use for treating lymphoma in a subject in need thereof. In another particular embodiment said subject is suffering from a B cell lymphoma. In another particular embodiment said subject is suffering from a Hodgkin or a non-Hodgkin B cell lymphoma. In another particular embodiment said subject is suffering from a B cell NHL. In another particular embodiment said subject is suffering from a B cell NHL selected from DLBCL, primary effusion lymphoma, mantle cell lymphoma, Burkitt's lymphoma, follicular lymphoma, B-LBL, preferably the B cell NHL is DLBCL.

As above-mentioned, the combinatory therapies of the invention lead to an unexpected strong induction of lymphoma cell death, making them particularly useful for preventing any resistance or metabolism reprogramming occurring during chronic treatments (*e.g*. metformin or chemotherapies). Hence, one object of the invention relates to the use of any of the combinatory therapy according to the invention to prevent metformin resistance and/or metabolism reprogramming upon chronic metformin therapy in a subject suffering from a lymphoma, in particular a Hodgkin or a non-Hodgkin B cell lymphoma. In another particular embodiment said subject is suffering from a B cell NHL. In another particular embodiment said subject is suffering from a B cell NHL selected from DLBCL, primary effusion lymphoma, mantle cell lymphoma, Burkitt's lymphoma, follicular lymphoma, B-LBL, preferably the B cell NHL is DLBCL.

Another object of the invention relates to the use of any of the combinatory therapy of the invention to combination to induce lymphoma cell death, including apoptosis, in a subject suffering from a lymphoma, in particular a Hodgkin or a non-Hodgkin B cell lymphoma. In a particular embodiment said subject is suffering from a B cell NHL. In another particular embodiment said subject is suffering from a B cell NHL selected from DLBCL, primary effusion lymphoma, mantle cell lymphoma, Burkitt's lymphoma, follicular lymphoma, B-LBL, preferably the B cell NHL is DLBCL.

A more particular object of the invention relates to the use of any of the combinatory therapy of the invention to combination to induce cytotoxicity in glycolytic and OXPHOS lymphoma cells in a subject suffering from a lymphoma, in particular a Hodgkin or a non-Hodgkin B cell lymphoma. In another particular embodiment said subject is suffering from a B cell NHL. In another particular embodiment said subject is suffering from a B cell NHL selected from DLBCL, primary effusion lymphoma, mantle cell lymphoma, Burkitt's lymphoma, follicular lymphoma, B-LBL, preferably the B cell NHL is DLBCL.

In an even more particular embodiment object of the invention relates to the use of any of the combinatory therapy of the invention to synergistically induce lymphoma cell death in a subject suffering from a lymphoma, in particular a Hodgkin or a non-Hodgkin B cell lymphoma. In a particular embodiment said subject is suffering from a B cell NHL. In another particular embodiment said subject is suffering from a B cell NHL selected from DLBCL, primary effusion lymphoma, mantle cell lymphoma, Burkitt's lymphoma, follicular lymphoma, B-LBL, preferably the B cell NHL is DLBCL. this strong induction of apoptotic process is a further advantage as it allows to expect an optimization of drug dosages in order to possibly reduce unwanted side effects. Also it could even lead to prevent development of resistance to compounds of the combinatory treatments of the invention when used alone (*e.g*. resistance to metformin) which is observed in some instances when used alone. Determining an actual synergistic effect from combinatory therapy of the invention is well known by the skilled in the art and further described in Foucquier and Guedj (2015) and can be carry on either *in vitro* on cultured lymphoma cells or *in vivo* in model animals.

Combinatory treatments according to the invention can be used either before or after an anticancer treatment such as chemotherapeutic treatment, a radiotherapy of lymphoma, and/or immune checkpoint inhibitors or carT cell therapy etc, in order to ensure a complete destruction of cancerous cells and thereby prevent or reduce the risk of relapse. Said chemotherapeutic treatments, , are known from those skilled in the art, they can be for example, and in a non-limitative way, for lymphomas: R-CHOP (mix of rituximab, cyclophosphamide, doxorubicin, vincristine, and prednisone), R-EPOCH (mix of rituximab, etoposide, cyclophosphamide, doxorubicin, vincristine and prednisone) or CHOP (mix of cyclophosphamide, doxorubicin, vincristine, and prednisone), CVP (cyclophosphamide, vincristine, prednisone), RDHAP (Rituximab, dexamethasone, cytarabine, cisplatin), CODOX-M (cyclophosphamide, vincristine [Oncovin], doxorubicin, and high-dose methotrexate), IVAC (ifosfamide, etoposide [VP-16], and cytarabine [ara-C]), R-ACVBP (doxorubicine (Adriblastine), cyclophosphamide, vindesine, bléomycine, prednisone) etc. Said immune checkpoint inhibitors are known from those skilled in the art, they can be for example, and in a non-limitative selected from nivolumab, pembrolizumab, atezolizumab, avelumab, durvalumab, cemiplimab, pidilizumab, AMP-224, AMP-514, PDR001, BMS-936559, CK-301.

The combinatory therapies are particularly efficient in a relapse and/or refractory subjects. Also, an object of the invention relates to any of the above-detailed combinatory therapy in a subject experiencing, or suspected to experience, a relapse for its use in treating a lymphoma, in particular a Hodgkin or a non-Hodgkin B cell lymphoma. In another particular embodiment said subject is suffering from a B cell NHL. In another particular embodiment, said subject is suffering from a B cell NHL selected from DLBCL, primary effusion lymphoma, mantle cell lymphoma, Burkitt's lymphoma, follicular lymphoma, B-LBL, preferably the B cell NHL is DLBCL.

Another object of the invention relates to any of the above detailed combinatory therapy in a subject experiencing or suspected to experience relapse for its use in slowing down or stopping progression of a lymphoma, in particular a Hodgkin or a non-Hodgkin B cell lymphoma. In another particular embodiment said subject is suffering from a B cell NHL. In another particular embodiment said subject is suffering from a B cell NHL selected from DLBCL, primary effusion lymphoma, mantle cell lymphoma, Burkitt's lymphoma, follicular lymphoma, B-LBL, preferably the B cell NHL is DLBCL.

Known treatments are for example radiotherapy and chemotherapeutic treatments (*e.g*. R-CHOP, R-EPOCH, CHOP, R-ACVBP). A particular object of the invention thus relates to any of the above detailed combinatory therapy in a subject found refractory to CHOP, R-CHOP or R-EPOCH treatment regimen for its use in treating a lymphoma, in particular a Hodgkin or a non-Hodgkin B cell lymphoma. In another particular embodiment said subject is suffering from a B cell NHL. In another particular embodiment said subject is suffering from a B cell NHL selected from DLBCL, primary effusion lymphoma, mantle cell lymphoma, Burkitt's lymphoma, follicular lymphoma, B-LBL, preferably the B cell NHL is DLBCL.

Combinatory treatments according to the invention exposed in any of paragraph [0033] to [0048] can be administered to the subject according different routes and regimen.

The administration of each drug comprised in the combinatory treatment according to the invention may be by any suitable means resulting in a concentration of the drug, when combined with the other at least one compound, able to ameliorate the patient condition or efficiently treat a lymphoma, preferably a Hodgkin or a non-Hodgkin B cell lymphoma, more preferably a B cell NHL, more preferably a B cell NHL selected from DLBCL, primary effusion lymphoma, mantle cell lymphoma, Burkitt's lymphoma, follicular lymphoma, B-LBL, and even more preferably the B cell NHL is DLBCL. As already mentioned, drugs comprised in the combination therapies of the invention can be administered concomitantly, either in the same or different pharmaceutical formulation, or sequentially. When administered sequentially, the time interval between the administration of each drugs or compounds comprised in the combinations according to the present invention should lead to strong induction of apoptosis and cell death, allowing the benefits of the efficient effect described herein. This time interval would be easily defined by the one skilled in the art *via* the PK/PD data of each of the compounds comprised in the combinations according to the present invention.

Each drugs or compounds comprised in the combinations according the invention can be administered in divided doses (*e.g*. two or three times or even more) or, preferably, as a single dose. Said divided or single doses can be administered daily, every other day, or every 3, 4 or 5 days or more, independently from one drug to another, for the duration on the treatment.

Related to cancer treatment, combinatory therapies of the invention are meant to be administered chronically, *e.g.* one to several times daily, for several days to several years. More preferably treatment combinatory therapy of the invention are meant to be administered chronically in cycles, as it is usual for chemotherapeutic treatments, in order to allow the organism to rest and avoiding potential side effects of drugs or compounds comprised in the combinatory treatment of the invention. Also, during these recovery periods, during which the subject is not administered with combinatory treatments of the invention, it might be desirable to administer another treatment in order to contain the development of the disease during this "off" period, especially in case of subject suffering from relapse and/or a refractory disease. In a cycle the period of administration of a combination therapy according to the invention can last from 1 day to 2, 3 , 4, 5 ,6, 7, 8, 9, 10, 11, 12, 11, 12, 13, 14, 15 days or even more; and the recovery period can last for 1 day to 2, 3, 4, 5, 6, 7, 8, 9, 10, 11, 12, 11, 12, 13, 14, 15 days or even more. Each of these periods being adapted, as it is already well established for current chemotherapeutic treatments of the art, as a function of the patient's condition.

Accordingly, a particular object of the invention relates to a cyclic administration, wherein each cycle comprises the followings:
a) the administration any of combinatory treatment as mentioned above every day for a 15 day period; and
b) a recovery period of 15 days.

Accordingly, a particular object of the invention relates to a cyclic administration, wherein each cycle comprises the followings:
a) the administration any of combinatory treatment as mentioned above every day for a 7 day period; and
b) a recovery period of 7 days.

Accordingly, a particular object of the invention relates to a cyclic administration, wherein each cycle comprises the followings:
a) the administration any of combinatory treatment as mentioned above every day for a 7 day period; and
b) a recovery period of 15 days.

Accordingly, a particular object of the invention relates to a cyclic administration, wherein each cycle comprises the followings:
a) the administration any of combinatory treatment as mentioned above every day for a 15 day period; and
b) a recovery period of 7 days.

Accordingly, a particular object of the invention relates to a cyclic administration, wherein each cycle comprises the followings:
a) the administration any of combinatory treatment as mentioned above every day for a 30 day period; and
b) a recovery period of 15 days.

Accordingly, a particular object of the invention relates to a cyclic administration, wherein each cycle comprises the followings:
a) the administration any of combinatory treatment as mentioned above every day for a 15 day period; and
b) a recovery period of 30 days.

Accordingly, a particular object of the invention relates to a cyclic administration, wherein each cycle comprises the followings:
a) the administration any of combinatory treatment as mentioned above every day for a 30 day period; and
b) a recovery period of 7 days.

Accordingly, a particular object of the invention relates to a cyclic administration, wherein each cycle comprises the followings:
a) the administration any of combinatory treatment as mentioned above every day for a 7 day period; and
b) a recovery period of 30 days.

In another particular embodiment of the invention, the cyclic administration is to be repeated as many times as needed for treating a subject in need thereof according to the present invention.

In a more particular object of the invention, said recovery period can comprises treating the subject with at least one compound or drug, which differs from the ones of the invention, aiming to contain the development of the disease. In a particular embodiment said at least one compound or drug to be administered during said recovery period is a mTOR inhibitor, more preferably a mTOR inhibitor selected from rapamycin and rapalogs as deferolimus, temsirolimus or everolimus, Consequently, a particular object of the invention relates to a combinatory treatment of the invention comprising at least one biguanide and at least one inhibitor of glutamine metabolism said combinatory treatment being administered chronically in cycles of a 15 days period of administration followed by a recovery period of 15 days during which a mTOR inhibitor, preferably selected from rapamycin or rapalogs as e.g., deferolimus, everolimus, temsirolimus is administered.

In a another particular embodiment, the invention relates to a combinatory treatment comprising at least an L-asparaginase as a glutamine inhibitor, wherein said L-asparaginase is administered on every other day, while the at least one inhibitor of the mitochondrial complex I of mitochondrial electron transport chain and/or at least one biguanide is administered on a daily basis in a subject in need thereof.

In a more particular embodiment, the invention related to a combinatory treatment comprising at least an L-asparaginase and metformin, or pharmaceutically acceptable salts, prodrugs, derivatives, or sustained release formulations thereof, wherein said L-asparaginase is administered on every other day, while metformin is administered on a daily basis in a subject in need thereof.

In an embodiment the at least one inhibitor of the mitochondrial electron transport chain is a biguanide selected from metformin, phenformin or buformine, or a biguanide having an inhibitory activity of complex I of mitochondrial electron transport chain in cells, and is administered orally at daily dosages of between 0.1 g and 7.5 g daily, more preferably between 0.5 g and 1.5 g daily, even more preferably 1 g daily. In another embodiment, the L-asparaginase is administered intravenously every other day at between 5000 and 30000 UI/m² in one hour, preferably between 10000 and 20000 UI/m² in one hour, even more preferably of the L-asparaginase is administered every other day at 15000UI/m² in one hour.

In a particular embodiment, in a combinatory treatment of the invention,
- metformin is administered orally at daily dosages of between 0.1 g and 7.5 g daily, more preferably between 0.5 g and 1.5 g daily, even more preferably 1 g daily, and
- an L-asparaginase is administered intravenously every other day at between 5000 and 30000 UI/m² in one hour, preferably between 10000 and 30000 UI/m² in one hour, even more preferably of the L-asparaginase is administered every other day at 15000 UI/m² in one hour,
   on a basis of cycles comprising a 15-days period during which the subject is administered with said metformin and L-asparaginase doses, and a 15-day recovery period comprising either no further lymphoma treatment or a another treatment able to content or to slow down disease development, for example a treatment based on mTOR inhibitors as exposed above. In case of relapse or B lymphoma resistant to CHOP, R-CHOP or R-EPOCH, mTOR inhibitors-based treatment is particularly preferred to be administered to the subject during recovery periods.

### Kits for Therapeutic methods and uses of the invention

Another object of the invention is a kit comprising:
- at least one inhibitor of the mitochondrial electron transport chain,
- at least one inhibitor of glutamine metabolism,, and
- instructions for its use in the treatment or the prevention of a lymphoma in a subject in need thereof, preferably a Hodgkin or a non-Hodgkin B cell lymphoma. In another particular embodiment, said subject is suffering from a B cell NHL. In another particular embodiment, said subject is suffering from a B cell NHL selected from DLBCL, primary effusion lymphoma, mantle cell lymphoma, Burkitt's lymphoma, follicular lymphoma, B-LBL, preferably the B cell NHL is DLBCL.

In a particular embodiment, said kit comprises:
- at least one inhibitor of the mitochondrial electron transport chain selected from metformin, phenformin or buformin ,
- at least one L-asparaginase, and
- instructions for its use in the treatment or the prevention of a lymphoma in a subject in need thereof, preferably a Hodgkin or a non-Hodgkin B cell lymphoma. In another particular embodiment said subject is suffering from a B cell NHL. In another particular embodiment said subject is suffering from a B cell NHL selected from DLBCL, primary effusion lymphoma, mantle cell lymphoma, Burkitt's lymphoma, follicular lymphoma, B-LBL, preferably the B cell NHL is DLBCL.

In a more particular embodiment, said kit comprises:
- metformin, or a pharmaceutically acceptable salt, prodrug, derivative, or sustained release formulation thereof
- at least one L-asparaginase a pharmaceutically acceptable salt, prodrug, derivative, or sustained release formulation thereof, and
- instructions for its use in the treatment or the prevention of a lymphoma in a subject in need thereof, preferably a Hodgkin or a non-Hodgkin B cell lymphoma. In another particular embodiment said subject is suffering from a B cell NHL. In another particular embodiment said subject is suffering from a B cell NHL selected from DLBCL, primary effusion lymphoma, mantle cell lymphoma, Burkitt's lymphoma, follicular lymphoma, B-LBL, preferably the B cell NHL is DLBCL.

In an even more particular embodiment said kit comprises:
- metformin, or a pharmaceutically acceptable salt, prodrug, derivative, or sustained release formulation thereof
- colaspase or crisantaspase, or a pharmaceutically acceptable salt, prodrug, derivative, or sustained release formulation thereof, and
- instructions for its use in the treatment or the prevention of a lymphoma in a subject in need thereof, preferably said subject is suffering from a Hodgkin or a non-Hodgkin B cell lymphoma. In another particular embodiment, said subject is suffering from a B cell NHL. In another particular embodiment, said subject is suffering from a B cell NHL selected from DLBCL, primary effusion lymphoma, mantle cell lymphoma, Burkitt's lymphoma, follicular lymphoma, B-LBL, preferably the B cell NHL is DLBCL.

In said kits according to the invention, the at least one inhibitor of the mitochondrial electron transport chain and the at least one inhibitor of glutamine metabolism can be packed either together or separately in a unit dosage form depending on specification of each compound. Advantageously, said kit can comprise the number of unit dosage forms suitable to do a full cycle of combinatory treatment of the invention as exposed above, *e.g.* as cycles specified in paragraphs [0075] to [0089].

For example, in a particular embodiment of the invention, the kit according to the invention further comprises the number of single doses needed to complete the administration cycle comprising:
a) the administration any of combinatory treatment as mentioned above every day for a 15 day period; and
b) a recovery period of 15 days.

Thus, said one kit can comprise:
- at least one inhibitor of the mitochondrial electron transport chain selected from metformin, phenformin or buformin, said at least one compound being packaged preferably in single doses to be administrated during the 15 day period;
- at least one inhibitor of glutamine metabolism, said at least one compound being preferably packaged in single doses to be administrated during the 15 day period;
- at least one mTOR inhibitor, said at least one compound being preferably packaged in single doses to be administrated during the 15 day period; and
- instructions for its use in the treatment or the prevention of a B-cell lymphoma in a subject in need thereof, preferably a Hodgkin or a non-Hodgkin B cell lymphoma. In another particular embodiment said subject is suffering from a B cell NHL. In another particular embodiment said subject is suffering from a B cell NHL selected from DLBCL, primary effusion lymphoma, mantle cell lymphoma, Burkitt's lymphoma, follicular lymphoma, B-LBL, preferably the B cell NHL is DLBCL.

### EXAMPLES

The following abbreviations have been used:
- DLBCL: diffuse large B-cell lymphoma
- COO: classification into cell-of-origin of DLBCL
- CCC: Consensus Clustering Classification (CCC) classification of tumours of DLBCL based on transcriptome clustering
- TMTV: total-body metabolic tumor volumes
- PET SCAN: Positron-emission tomography scanner exam
- Oxphos: CCC tumour subtype mainly reliant on oxidative phosphorylation
- BCR: CCC tumour subtype mainly reliant on glycolysis
- GCB or GC: COO DLBCL tumour subtype
- ABC: COO DLBCL tumour subtype
- Type 3: COO DLBCL tumour subtype

The following examples illustrate the invention without limiting its scope in any way.

### 1. Materials and methods

### Human DLBCL cell lines

The DLBCL cell lines MD901, OCI-LY19, SU-DHL4, SU-DHL6, Karpas422 (K422), HBL-1, PFEIFFER, U2932 and TOLEDO were obtained in collaboration with Jose Angel Martinez-Climent from Centro de Investigación Medica Aplicada (CIMA, <http://www.cima.es>) (Pamplona, Spain). Two classifications are considered in the art. The first is the classification into cell-of-origin (COO) subtypes, which is based on the identification of phenotypic subgroups of DLBCL sharing similarities with different stages of differentiation of B lymphocytes. These groups are: Germinal Center B cell (GCB), Activated B cell (ABC), cells being not GCB or ABC, are categorized as unclassified (type 3). The other, Consensus Clustering Classification (CCC) is based on the tumor transcriptome clustering and defines BCR (DLBCLs with elevated expression of BCR signalling components), oxidative phosphorylation (OxPhos, with elevated expression of genes involved in oxidative phosphorylation and mitochondrial metabolism) and host response (HR, enriched in genes associated with tumor microenvironment and host inflammatory response). BCR-DLBCLs have greater glycolytic flux typical of the Warburg phenotype whereas OxPhos-DLBCLs channel the majority of glucose-derived pyruvate into mitochondria, display elevated mitochondrial electron transport chain (ETC) activity, ATP production, and fatty acid oxidation (FAO) (Illana A. Stanley. 2014).

**Table 2**

| **DLBCL cell lines** | **Classification** | |
|---|---|---|
| | **COO** | **CCC** |
| MD901 | ABC | unknown |
| U2932 | ABC | BCR |
| HBL-1 | ABC | BCR |
| TOLEDO | TYPE 3 | OxPhos |
| PFEIFFER | TYPE 3 | OxPhos |
| K422 | GCB | OxPhos |
| SUDHL4 | GCB | BCR |
| SUDHL6 | GCB | BCR |
| OCI-LY19 | GCB | OxPhos |

### Cell culture

Cells were grown in RPMI-1640 Glutamax medium (#61870010, Thermo Fisher Scientific) enriched with 10% Fetal Bovine Serum (Hyclone), 100 U/mL penicillin-streptomycin at 37 °C in an incubator with a humidified atmosphere of 5% CO₂.

### Compounds

Metformin hydrochloride (PHR1084, Sigma-Aldrich/Merck KGaA, St. Louis, USA) was diluted in distilled filtered water to a final stock solution of 250 mM. Kidrolase (colaspase, Jazz Pharmaceuticals, Dublin, Ireland) was diluted in distilled filtered water to a final stock solution of 1.000 UI/ml. Rapamycin (553211, Calbiochem/Merck KGaA, St. Louis, USA) was diluted in DMSO to a final stock solution of 10 µM.

### Flow cytometry analysis: annexin V binding assay and mitochondrial transmembrane potential

Apoptotic cells were detected using Annexin V binding assay. Briefly after incubation, 10⁵ cells/mL were harvested, washed twice with cold PBS, suspended in 1X binding buffer and stained with Annexin V/ Phycoerythrin (PE) (BD Biosciences Pharmingen) and 4',6-diaminidino-2-phenylindole (DAPI, Molecular Probes) for 15 min at room temperature in the dark. Cells stained Annexin V+/PE-, and Annexin V+/PE+ were considered apoptotic, excluding DAPI-positive cells from gating. The samples were subjected to cytometric analysis with a MACSQuant Analyzer 10 Flow Cytometer (Miltenyi Biotec) and the data was statistically evaluated using the Flowjo v10.2 software. Synergy was determined according to Bliss (1939).

For determination of mitochondrial transmembrane potential (ΔΨm), 105 cells/mL cells were stained with 100 nM of Tetramethyl Rhodamine Methyl Ester (TMRM) (Molecular Probes) in RPMI-1640 Glutamax medium for 30 min at room temperature. Similarly, the analyses were evaluated by cytometry with MACSQuant Analyzer 10 Flow Cytometer (Miltenyi Biotec).

### Protein extracts

The cells are washed in cold 1X PBS then lysis is carried out at 4°C (on ice) for 20 minutes, using a mix containing a High Salt buffer (0.4 M NaCl). 25 mM Hepes pH 7.7, 1.5 mM MgCI 2, 0.2 mM EDTA, 1%NP4O), protease and phosphatase inhibitors (20 mM glycerol phosphate, 0.2 mM Na3VO4, 10mM PNPP, 2 mM DTT, 0.1 M PMSF and an inhibitor cocktail from Boehringer Company). Whole cell extracts are centrifuged also at 4°C for 10 minutes at 13 000 rpm, the supernatant is transferred to an Eppendorf tube and the proteins are dosed using a protein kit assay (Bio-Rad) and stored at - 80°C.

### Immunoblotting

Immunoblotting was performed as previously reported (Authier H et al, 2014). Cleaved caspase 3 and β-actin antibodies are from Cell Signaling Technologies and Sigma, respectively.

### Glutamine starvation

For experiments based on glutamine starvation, specific media were prepared as follows: 1) Control medium: RPMI 1640 with D-glucose, L-Glutamine, 1% penicillin-streptomycin, 10% FBS. 2) Glutamine deprived medium: RPMI 1640 without glutamine, with 1% penicillin-streptomycin, 10% FBS and D-Glucose. Cells were seeded at a density of 10⁶ cells/mL with the specific medium starvation (control, glucose deprived and glutamine deprived media) in a 12-wells plate.

For each condition, cell growth and cell viability were evaluated at 24h, 48h and 72h by Trypan Blue dye exclusion.

### Treatment of a refractory patient with combinatorial treatment according to the invention

A patient suffering from a DLBCL refractory to R-CHOP was administered with the combinatory treatment of the invention in compliance with legal provisions.

A combinatory treatment of the invention comprising metformin and L-asparaginase was administered as cycles comprising 15 days of treatment followed by 15 days of recovery. On the first 15 days, patient was administered with metformin at a daily dosage of 1000 mg of metformin and L-asparaginase (Kidrolase) infused at 10 000 UI/m² on 1 hour every other day. Blood samples and lymph nodes biopsies were taken from patient to follow the progress of the disease.

Blood LDH levels were dosed using 7D69 Lactate Dehydrogenase Reagent Kit (Abbott) and Architect c System.

Lymph nodes slides from biopsies performed at day 0 and to day 1 of cycle 1 were coloured with hematoxylin and eosin (HE).

Patient also underwent ¹⁸FDG PET-CT and measurement of Total Metabolic Tumour Volume (TMTV) on a regular basis to follow progression of the disease (Vercellino L et al. 2020).

### 2. Results

### DLBCL cells critically rely on Glutamine for their survival, independently of their COO or CCC classification.

Inventors have been able to identify for the first time that glutamine is essential for the survival of B lymphoma cells, especially DLBCL cells. As shown on Figure 1, glutamine starvation induces cell death in all the tested cells lines encompassing all the subtypes of COO or CCC classification. Hence, it is evidence that these cells depend on glutamine for their growth and division, independently from their cell of origin or metabolic class.

### Combinations according to the invention induces important apoptosis in B cell lymphoma cells.

### Caspase 3 activation upon treatment with combination of the invention

Caspase 3 is a protease known to be associated with the initiation of the "death cascade" and is considered as a marker of the cell's entry point into the apoptotic signalling pathway. Caspases are synthesised as inactive pro-caspases, and their activation implies dimerization of procaspases following by their cleavage.

As shown in Figure 2B, while activation of caspase 3 is not to barely detectable using either metformin or L-asparaginase, a very important increase in cleaved caspase 3 in both ABC (MD901 cell line) and GCB (OCI-LY19 cell line) cell types treated with the combination of the two drugs.

### Phosphatidylserine relocalization

In the early phase of apoptosis, the translocation of phosphatidylserine is observed from the inner leaflet of the plasma membrane to the outside which is highlighted by specific fixation of annexin V on phosphatidylserine. As above-mentioned the combinatory treatment according to the invention is found effective in massive induction of apoptosis in B lymphoma cells (Figure 2A). Of note, a strong induction of apoptosis is measured for cells treated with the combination of metformin and L-asparaginase in a significantly greater amplitude in comparison with metformin or L-asparaginase when used alone. This greater effect is observed from 24h of incubation with the drug and last up to 3 days of incubation with the combination of metformin and L-asparaginase. More importantly, such massive induction of apoptosis is observed in Oxphos and BCR CCC type cells. In other words, combination of metformin and L-asparaginase efficiently induces apoptosis independently of the metabolic type of the tumour (Figure 2 and summarized in table 3 below). Also, combination of metformin and L-asparaginase statistically potentiates the induction of cell apoptosis

**Table 3**

| | Cell lines | | |
|---|---|---|---|
| | SUDHL4 | OCI-LY19 | MD901 |
| CCC type | GCB | GCB | ABC |
| COO type | Oxphos | BCR | unknown |
| Synergistic induction of apoptosis | yes | yes | yes |

### Collapse of transmembrane potential

In the succession of events taking part in apoptosis, mitochondria loss of integrity usually past the point of no return. The ΔΨm collapse leads to the opening of the mitochondrial permeability transition pores within the mitochondrial membrane thereby resulting in the leaking of cytochrome C in cytosol. A significantly stronger loss is observed for B lymphoma cells when treated with a combination of metformin and L-asparaginase in comparison with the drug administered alone (not shown).

### Conclusion

Hence, results provided herein show that combinatory treatment of the invention displays an ubiquitous apoptotic effect regardless of the metabolic type of B cell lymphoma. Moreover, such effect is also observed regardless of the COO type of DLBCL cells. Furthermore, this strong induction of apoptotic process by a combinatory treatment of the invention is also found to be synergistic which is a further advantage as it allows to expect an optimization of drug dosages in order to possibly reduce unwanted side effects. This could even lead to prevent development of resistance to metformin which is observed in some instances when used alone.

### mTOR inhibitors antagonize compounds of combinatory treatments of the invention.

As shown in Figure 3 rapamycin antagonizes apoptotic effect of metformin or L-asparaginase in DLBCL cell lines. Hence, while mTOR inhibitors could be seen as of use to be further added to the combinatory treatments of the invention, results provided herein show that coadministration of said drugs should be avoided. Nevertheless, mTOR inhibitors could be useful during the recovery period of a cycle of a combinatory treatment of the invention, in order to control disease progression during this period. Indeed, no negative impact of rapamycin treatment on apoptosis induction or cancer cell death was observed in an experimental set up using a sequential incubation of MD901 cells with first a combination of metformin and L-asparaginase and, second, 24 hours later, for 24h, a treatment with rapamycin (20 nM).

### Combinatory treatments of the invention induce progressive remission in relapsed/refractory patient with B lymphoma.

A patient suffering from a relapse and refractory DLBCL was treated with a combinatory treatment of the invention comprising metformin and L-asparaginase as explained in the materials and methods section. A progressive remission was observed in this patient who experienced a significant increase of his life duration expectancy (over 6 months). Signs of progressive remission were observed from the first day of combinatory treatment administration. Figure 4A shows a marked induction of necrosis of cancer cells in lymph node biopsy observed after the first day of treatment. These results being confirmed by the lowering in LDH plasmatic dosage reported in table 4 below.

**Table 4**

| | Cycle 1 | Cycle 2 | |
|---|---|---|---|
| | Day 1 | Day 1 | Day 15 |
| Plasmatic LDH level (UI/L) | 544 | 435 | 329 |

Effectiveness of the tested combination was embodied by the massive reduction of TMTV (69%) which was observed as soon as the end of cycle 2 of the combinatory treatment (Figure 4B).

### Conclusion

Combinatory treatments of the invention are able to induce progressive remission in patients suffering from lymphomas, and especially in relapse and/or refractory patients.

### 3. Conclusion

Results provided herein show an important and unexpected increase in cancer cells death upon combinatory treatment comprising at least one inhibitor of mitochondrial electron transport chain and at least one inhibitor of glutamine metabolism, regardless of the CCC or COO subtype of the tumour. Such ubiquitous effectiveness opens up new possibilities for patients, especially those experiencing relapse or refractoriness to current treatments.

### REFERENCES

Authier H, Billot K, Derudder E, Bordereaux D, Rivière P, Rodrigues-Ferreira S, Nahmias C, and Baud V. IKK phosphorylates RelB to modulate its promoter specificity and promote fibroblast migration downstream of TNF receptors. PNAS 2014 Oct. 14 111 (41) 14794-14799.
Bliss Cl, The toxicity of poisons applied jointly. Annals of Applied Biology 1939, 26: 585-615. <doi:10.1111/j.1744-7348.1939.tb06990.x>
Chiche J, Reverso-Meinietti J, Mouchotte A, Rubio-Patiño C, Mhaidly R, Villa E, Bossowski JP1, Proics E, Grima-Reyes M, Paquet A, Fragaki K, Marchetti S, Briere J, Ambrosetti D, Michiels JF, Molina TJ, Copie-Bergman C, Lehmann-Che J, Peyrottes I, Peyrade F, de Kerviler E, Taillan B, Garnier G, Verhoeyen E, Paquis-Flucklinger V, Shintu L, Delwail V, Delpech-Debiais C, Delarue R, Bosly A, Petrella T, Brisou G, Nadel B, Barbry P, Mounier N, Thieblemont C, Ricci JE. GAPDH Expression Predicts the Response to R-CHOP, the Tumor Metabolic Status, and the Response of DLBCL Patients to Metabolic Inhibitors. Cell Metab. 2019 Jun 4;29(6):1243-1257.
Foucquier J, Guedj M. Analysis of drug combinations: current methodological landscape. Pharmacol Res Perspect. 2015;3(3)
Stanley, Illana Allake. 2014. Metabolic Heterogeneity in Molecular Subsets of Diffuse Large B-cell Lymphoma. Doctoral dissertation, Harvard University. Available at : <https://dash.harvard.edu/handle/1/13069713>.
Vercellino L, Cottereau AS, Casasnovas RO, Tilly H, Feugier P, Chartier L, Fruchart C, Roulin L, Obéric L, Pica GM, Ribrag V, Abraham J, Simon M, Gonzalez H, Bouabdallah R, Fitoussi O, Sebban CJ, Lopez-Guillermo A, Sanhes L, Morschhauser F, Trotman J, Corront B, Choufi B, Snauwaert S, Godmer P, Briere J, Salles GA, Gaulard P, Meignan M, Thieblemont C. High total metabolic tumor volume at baseline allows discrimination of survival even in patients aged 60 to 80 years responding to R-CHOP. Blood. 2020 Jan 24. blood.2019003526. doi: <https://doi.org/10.1182/blood.2019003526>

## Claims

1. A combination comprising at least one inhibitor of glutamine metabolism or a pharmaceutically acceptable salt, prodrug, derivative, or sustained release formulation thereof and at least one inhibitor of mitochondrial electron transport chain or a pharmaceutically acceptable salt, prodrug, derivative, or sustained release formulation thereof, for its use in the treatment or the prevention of a lymphoma in a subject in need thereof.

2. The combination for its use according to claim 1, wherein the at least one inhibitor of glutamine metabolism is selected from telaglenastat, an L-asparaginase, gamma-l-Glutamyl-p-Nitroanilide, (2S)-2-amino-3-phenylmethoxypropanoic acid, 2-phenyl-N-[5-[2-[2-[5-[(2-phenylacetyl)amino]-1,3,4-thiadiazol-2-yl]ethylsulfanyl]ethyl]-1,3,4-thiadiazol-2-yl]acetamide, (2S)-2-amino-6-diazo-5-oxohexanoic acid, 5-[3-bromo-4-(dimethylamino)phenyl]-2,3,5,6-tetrahydro-2,2-dimethyl-benzo[a]phenanthridin-4(1H)-one, 5-(2-Propoxyphenyl)-1H-[1,2,3]triazolo[4,5-d]pyrimidin-7(4H)-one, or 2,2-Dimethyl-beta-alanine or pharmaceutically acceptable salts, prodrugs, derivatives, or sustained release formulations thereof, preferably colaspase or crisantaspase or pharmaceutically acceptable salts, prodrugs, derivatives, or sustained release formulations thereof.

3. The combination for its use according to anyone of claims 1 or 2, wherein the at least one inhibitor of mitochondrial electron transport chain is selected from metformin, phenformin, buformin, (2R,6aS,12aS)-1,2,6,6a,12,12a-hexahydro-2-isopropenyl-8,9-dimethoxychromeno[3,4-b]furo(2,3-h)chromen-6-one, [(1R,2R,6R,10S,11R,13S,15R,17R)-13-benzyl-6-hydroxy-4,17-dimethyl-5-oxo-15-prop-1-en-2-yl-12,14,18-trioxapentacyclo[11.4.1.01,10.02,6.011,15]octadeca-3,8-dien-8-yl]methyl 2-(4-hydroxy-3-methoxyphenyl)acetate, (E)-N-[(4-hydroxy-3-methoxyphenyl)methyl]-8-methylnon-6-enamide, [(2*R*,3*R*)-5,7-dihydroxy-2-(3,4,5-trihydroxyphenyl)-3,4-dihydro-2*H*-chromen-3-yl] 3,4,5-trihydroxybenzoate, (3R,4S)-3,4-bis(4-hydroxyphenyl)-8-methyl-3,4-dihydro-2H-chromen-7-ol, elesclomol, 6-methyl-N-[(E)-1-pyridin-2-ylethylideneamino]-4a,5-dihydro-2H-[1,2,4]triazino[5,6-b]indol-3-amine or pharmaceutically acceptable salts, prodrugs, derivatives, or sustained release formulations thereof, preferably metformin or a pharmaceutically acceptable salts, prodrugs, derivatives, or sustained release formulations thereof.

4. The combination for its use according to any one of claims 1 to 3, wherein the at least one inhibitor of glutamine metabolism is a L-asparaginase or a pharmaceutically acceptable salts, prodrugs, derivatives, or sustained release formulations thereof and the at least one inhibitor of mitochondrial electron transport chain is selected from metformin, phenformin or buformin or a pharmaceutically acceptable salts, prodrugs, derivatives, or sustained release formulations thereof.

5. The combination for its use according to any one of claims 1 to 4, wherein the compounds are formulated for a combined or separate administration.

6. The combination for its use according to any one of claim 1 to 5, wherein the compounds are administered separately at the same time or sequentially.

7. The combination for use according to anyone of claims 1 to 6, wherein the lymphoma is a Hodgkin or a Non-Hodgkin lymphoma (NHL), preferably said lymphoma is a B-cell lymphoma, preferentially a B-cell NHL selected from diffuse large B-cell lymphoma (DLBCL), primary effusion lymphoma, mantle cell lymphoma, Burkitt's lymphoma, follicular lymphoma, precursor B-cell lymphoblastic leukemia/lymphoma (B-LBL), and more preferably DLBCL.

8. The combination for its use according to any one of claims 1 to 7, wherein the at least least one inhibitor of glutamine metabolism is at least one L-asparaginase or a pharmaceutically acceptable salt, prodrug, derivative thereof, formulated to be infused every other day from 5000 to 30000 UI/m² in one hour, from 10000 UI/m² to 20000 UI/m² in one hour, preferably 15000UI/m² in one hour.

9. The combination for its use according to any one of claims 1 to 8, wherein the at least one inhibitor of the mitochondrial electron transport chain is a biguanide selected from metformin, buformin or phenformin, or a pharmaceutically acceptable salt, prodrug, derivative thereof and is administered from 0.1 g to 7.5 g daily, preferably from 0.5 g to 1.5 g daily.

10. The combination for its use according to anyone of claims 1 to 9, wherein said combination is administered in cycles, each cycle comprising:
- a first period during which said combination is administered to the subject, and
- a second period of recovery during which said combination is not administered to the subject.

11. The combination for its use according to claim 10, wherein at least one mTOR inhibitor is administered to said subject during the recovery period, said mTOR inhibitor being preferably selected from the group consisting in: rapamycin and rapalogs as deferolimus, everolimus, temsirolimus.

12. The combination for its use according to any one of claims 1 to 11, wherein said combination is administered before and/or after a chemotherapeutic treatment and/or a radiotherapy and/or a graft and/or an immune checkpoint inhibitor treatment.

13. The combination for its use according to any one of claim 12 wherein the lymphoma is DLBCL and the chemotherapeutic treatment is based on a regimen selected from CHOP, R-CHOP or R-EPOCH, R-ACVBP.

14. The combination for its use according to any one of claims 1 to 11, wherein said subject is relapse or refractory to chemotherapy and/or graft and/or checkpoint inhibitor therapy and/or radiotherapy.

15. The combination for its use according to anyone of claims 1 to 14 wherein said combination induces cytotoxicity in both glycolytic and OXPHOS type cancer cells from lymphoma.

16. The combination for its use according to anyone of claims 1 to 15 wherein said combination prevents resistance of cancer cells to metformin treatment.
